# EUROPÄISCHE PATENTANMELDUNG

(11) **EP 0 719 784 A2**
(43) Veröffentlichungstag der Anmeldung: **03.07.1996**
(21) Anmeldenummer: 95116016.7
(22) Anmeldetag: 11.10.1995
(51) Int. Cl.: C07H 15/04

(54) **Verfahren zur Bleichung von Alkylpolyglycosiden**

(30) Priorität: 03.12.1994 DE 4443084
(71) Anmelder: HÜLS AKTIENGESELLSCHAFT, D-45764 Marl (DE)
(72) Erfinder: Grützke, Jürgen, D-44803 Bochum (DE); Schmidt, Stefan, Dr., D-45721 Haltern (DE)

(57) **Zusammenfassung**

Die Erfindung betrifft ein Verfahren zur Bleichung von wasser- und fettalkoholarme Alkylpolyglycosiden mit festen Oxidationsmitteln unter Durchmischung insbesondere in einem Kneter oder in einem Extruder bei Temperaturen von 80 bis 160 °C.

## Beschreibung

Die Erfindung betrifft ein Verfahren zur Bleichung von wasser- und fettalkoholarmen Alkylpolyglycosiden mit festen Oxidationsmitteln unter Durchmischung bei Temperaturen im Bereich von 80 bis 160 °C. Die Durchmischung erfolgt z. B. in einem Kneter oder in einem Extruder.

Alkylpolyglycoside werden aus natürlichen Rohstoffen hergestellt und sind ungiftige und leicht abbaubare oberflächenaktive Stoffe. Sie werden deshalb als Wasch- und Reinigungsmittel und als Emulgatoren und Dispergatoren verwendet. Sie haben aber nur dann die gewünschten Grenzflächeneigenschaften, wenn die Alkylgruppen mindestens 8 Kohlenstoffatome aufweisen. Die Alkylpolyglycoside im Sinne dieser Erfindung genügen der Formel

R' - O - Zₙ,

in der R' für einen unverzweigten oder verzweigten, gesättigten oder ungesättigten aliphatischen Alkylrest mit 8 bis 18 Kohlenstoffatomen oder Gemische davon und Zₙ für einen Polyglycosylrest mit n = 1,1 bis 3 Hexose- oder Pentoseeinheiten oder Gemische davon stehen.

Bevorzugt werden Alkylpolyglycoside mit Alkylresten mit 12 bis 16 Kohlenstoffatomen sowie einem Polyglycosylrest mit n = 1,1 bis 2. Besonders bevorzugt werden Alkylpolyglycoside mit einem Polyglycosylrest von n = 1,1 bis 1,5 und ganz besonders bevorzugt von n = 1,2 bis 1,4. Als Polyglycosylrest wird der Alkylpolyglucosylrest bevorzugt. Alkylpolyglycoside mit langkettigen Alkylgruppen werden im allgemeinen durch ein- oder mehrstufige Synthesen hergestellt. Ein einstufiges Herstellungsverfahren wird u. a. in DE-A-41 01 252 beschrieben.

Ein zweistufiges Herstellungsverfahren wird beispielsweise in EP-A-0 306 652 angegeben, wonach man zunächst durch Glycosidierung mit n-Butanol ein n-Butylglycosid und darauf durch Umglycosidierung mit einem langkettigen Alkohol das gewünschte langkettige Alkylpolyglycosid herstellt.

Nach beendeter Reaktion liegen die Alkylpolyglycoside in langkettigen Alkoholen gelöst vor. Anschließend müssen diese Alkohole abgetrennt werden, wenn man in Wasser klar lösliche Produkte erhalten will. Alkylpolyglycoside sind Produkte, die bei höheren ästhetischen Anforderungen einer Bleichreaktion unterworfen werden müssen, damit man zu ausreichend hellen Produkten gelangt. Diese Bleichung wird meistens mit wäßrigen Alkylpolyglycosid-Lösungen unter Verwendung von Wasserstoffperoxid durchgeführt. Nachteilig sind bei diesem Verfahren die schlechten Raum-Zeit-Ausbeuten, vor allem, wenn zum vollständigen Abbau des Restperoxids die wäßrigen Alkylpolyglycosid-Lösungen noch längere Zeit auf erhöhter Temperatur gehalten werden müssen. Dadurch kann das Produkt geschädigt werden, wobei dunkle Farben sowie Zersetzungsprodukte mit z. T. unangenehmen Geruch auftreten. Außerdem besteht die Gefahr einer nicht mehr beherrschbaren Schaumentwicklung.

Die WO 91/19723 beschreibt einen Weg zur Alkylpolyglycosid-Bleichung, wobei der praktisch fettalkohol- und wasserfreie Stoff bei Temperaturen von 50 - 150 °C mit wäßrigem Wasserstoffperoxid und z. B. Natronlauge als Base verknetet wird. Die oben beschriebenen Nachteile der Alkylpolyglycosid-Bleichung in wäßriger Lösung treten bei dieser Bleichmethode nicht mehr auf. Nachteilig ist, daß die Bleichung bei Temperaturen nennenswert über 100 °C auf diese Weise nicht möglich ist. Fettalkohol- bzw. wasserarme Produkte sind aber häufig erst bei Temperaturen von über 100 °C knetbar. Schwierigkeiten können außerdem bei der Natronlauge-Zudosierung auftreten. Durch die Zugabe an Base muß ein gewisser Säuerungseffekt während der Bleichreaktion kompensiert werden. Eine geregelte Zugabe der Natronlauge wäre nur mit großem Aufwand zu realisieren, weil der pH-Wert nicht direkt in der Schmelze gemessen werden kann. Eine Zudosierung von Hand bringt immer die Gefahr der Über- oder Unterdosierung, wodurch sich das Bleichergebnis rapide verschlechtern würde.

Es bestand daher die Aufgabe, ein Verfahren zu finden, daß es erlaubt, die oxidierende Bleichung in einer Schmelze ohne die geschilderten Probleme mit der Dosierung von Wasserstoffperoxid und Natronlauge durchzuführen.

Es wurde nun gefunden, daß auch bei Temperaturen über 100 °C sehr gute Bleichergebnisse erzielt werden können, wenn statt der wäßrigen Wasserstoffperoxidlösung feste Bleichmittel wie Natriumpercarbonat oder Natriumperborat verwendet werden.

Gegenstand der Erfindung ist daher ein Verfahren zur Bleichung von wasser- und fettalkoholarmen Alkylpolyglycosiden, das dadurch gekennzeichnet ist, daß feste Oxidationsmittel hinzugefügt werden und die Reaktion unter Durchmischung bei Temperaturen von 80 bis 160 °C durchgeführt wird.

Dabei werden bevorzugt Temperaturen von 90 bis 150 °C und ganz besonders bevorzugt Temperaturen von 100 bis 140 °C angewendet. Als Alkylpolyglycosid wird vorzugsweise das Alkylpolyglucosid eingesetzt.

Die Stoffe lassen sich ohne Schwierigkeiten mit Hilfe von z. B. Extrudern oder Knetmaschinen in zähflüssiges APG (Temp. 80 - 160 °C) einarbeiten, so daß homogene Produkte resultieren. Eine Zugabe von Alkali während der Bleichung erübrigt sich, weil bei Verwendung dieser Bleichmittel keinerlei pH-Wert-Absenkung zu verzeichnen ist. Die erhaltenen Produkte sind sehr hell und ausgesprochen geruchsarm. Durch Zusatz kleiner Mengen Wasser vor der Bleichung kann das Resultat weiter verbessert werden. Der Zusatz an Wasser kann 0 bis 10 % betragen, vorzugsweise liegt er jedoch zwischen 0 und 5 %. Der Fettalkoholgehalt bei der Bleichung beträgt 0 bis 10 %, vorzugsweise 0 bis 5 %, wobei Werte von 0 bis 2,5 % ganz besonders bevorzugt werden.

## Patentansprüche

1. Verfahren zur Bleichung von wasser- und fettalkoholarmen Alkylpolyglycosiden, dadurch gekennzeichnet, daß feste Oxidationsmittel hinzugefügt werden und die Reaktion unter Durchmischung bei Temperaturen von 80 bis 160 °C durchgeführt wird.

2. Verfahren zur Bleichung von wasser- und fettalkoholarmen Alkylpolyglycosiden nach Anspruch 1, dadurch gekennzeichnet, daß als festes Oxidationsmittel Natriumpercarbonat verwendet wird.

3. Verfahren zur Bleichung von wasser- und fettalkoholarmen Alkylpolyglycosiden nach Anspruch 1, dadurch gekennzeichnet, daß als festes Oxidationsmittel Natriumperborat verwendet wird.

4. Verfahren zur Bleichung von wasser- und fettalkoholarmen Alkylpolyglycosiden nach einem der vorhergehenden Ansprüche, dadurch gekennzeichnet, daß die Reaktion bei Temperaturen von 90 bis 150 °C durchgeführt wird.

5. Verfahren zur Bleichung von wasser- und fettalkoholarmen Alkylpolyglycosiden nach einem der vorhergehenden Ansprüche, dadurch gekennzeichnet, daß die Reaktion bei Temperaturen von 100 bis 140 °C durchgeführt wird.

6. Verfahren zur Bleichung von wasser- und fettalkoholarmen Alkylpolyglycosiden nach einem der vorhergehenden Ansprüche, dadurch gekennzeichnet, daS 0 bis 5 % Wasser zugesetzt wird.

7. Verfahren zur Bleichung von wasser- und fettalkoholarmen Alkylpolyglycosiden nach einem der vorhergehenden Ansprüche, dadurch gekennzeichnet, daß das Alkylpolyglycosid ein Alkylpolyglucosid ist.

8. Verfahren zur Bleichung von wasser- und fettalkoholarmen Alkylpolyglycosiden nach einem der vorhergehenden Ansprüche, dadurch gekennzeichnet, daß die Durchmischung in einem Kneter erfolgt.

9. Verfahren zur Bleichung von wasser- und fettalkoholarmen Alkylpolyglycosiden nach einem der vorhergehenden Ansprüche, dadurch gekennzeichnet, daß die Durchmischung in einem Extruder erfolgt.
